# EUROPEAN PATENT APPLICATION

(11) **EP 2 804 117 A2**
(43) Date of publication of application: **19.11.2014**
(21) Application number: 14020060.1
(22) Date of filing: 16.05.2014
(51) Int. Cl.: G06F 19/00

(54) **Method and system for image report interaction for medical image software**

(30) Priority: 17.05.2013 US 201361824408 P; 15.05.2014 US 201414278580
(71) Applicant: Algotec Systems Ltd., 43107 Raanana (IL)
(72) Inventor: Shreiber, Reuven R., 34366 Haifa (IL)
(74) Representative: Carstens, Dirk Wilhelm

(57) **Abstract**

A system and method for image based report correction for medical image software, which incorporates such report correction as part of the report generation process. Such a system and method features a report generator, a report correction functionality and also some type of medical image software, for providing medical image processing capabilities, which allows the doctor or other medical personnel to generate the report, and as part of the report generation process, to be checked by the report correction functionality.

## Description

### FIELD OF THE INVENTION

The present invention relates to a system and method for image and report interaction workflow for medical image software and particularly, but not exclusively, to incorporating image based report corrections as part of the report generation process.

### BACKGROUND OF THE INVENTION

Medical image software has become an increasingly important diagnostic tool. Such software allows skilled medical personnel, such as doctors, to view, manipulate and interact with medical images such as CT (computerized tomography) scans, MRI (magnetic resonance imaging) scans, PET (positron emission tomography) scans, mammography scans and the like. As the amount of information that radiologists are forced to handle increases, so is the time spent on each study. In addition, the number of studies a radiologist needs to review is increasing as well. This causes a severe bottleneck in interpreting and reporting studies for further follow-up by the referring physicians. Radiologists produce reports that are later distributed to the referring physicians. As the amount of work that radiologists are forced to deal with increases, more errors find themselves into the reports. Many of these errors are caused by minor oversights, however their effect on patient care can be profound. Therefore, doctors need to be able to accurately and rapidly interact with medical image processing software and ultimately, to be able to report and share their results in as short and efficient a time as possible, in as error free a manner as possible, so as to speed up patient care.

Report generation occurs while the radiologist or other trained personnel is working with dedicated reporting software. This software is usually installed on a radiology reporting station in parallel to the review software (such as a PACS [Picture Archiving And Communication System] viewer or dedicated workstation) or integrated into the PACS viewer itself. For example, the latter type of installation may be found for native reporting on Carestream's Vue PACS. Present case scenarios for radiology reporting do not involve any connection between the images viewed and the report being dictated. Although the radiologist often dictates the report while examining the images, no interaction between the image viewing and report generation software occurs.

Any type of report generation may lead to errors, simply due to physician fatigue or inattention, such as for example when the physician indicates that an image shows an injury on the right side while in fact the injury is found on the left side. Such errors can lead to serious diagnostic mistakes.

### SUMMARY OF THE INVENTION

The present invention, in at least some embodiments, provides a system and method for allowing a report generation module to interact with an image viewing module. This interaction enables the radiologist to generate reports that are more precise and less prone to errors. Such a system and method features a report generator, a report parsing capability, an image interaction module and also some type of medical image software for viewing images with which the image interaction module interacts with. The combination of such modules allows the doctor or other medical personnel to generate the report, and as part of the report generation process, to support the image based detection and correction of errors.

The present invention, in at least some embodiments, supports this operation by having portions of the image visually indicated, for example by being highlighted or marked, according to words appearing in the report. For example, suppose that the radiologist is dictating a report on the reporting module and, as part of the image review process, detects a fracture on the left side of the head. During the dictation of the report, the radiologist makes a mistake and dictates that a fracture appears on the right side of the head. The present invention, in at least some embodiments, parses the text being dictated for keywords indicating location. It notes that the radiologist dictated the word "right" and consequently highlights the right part of the head. The radiologist then sees that the right side of the head is being highlighted, while the fracture he intended to report on is on the non-highlighted part in the left side of the head. He consequently then realizes his mistake and corrects it to "left". The present invention, in at least some embodiments, then recognizes the word "left" and highlights the left part of the image. The radiologist then receives positive feedback for his reporting in the image when the area containing the fracture is highlighted in the image.

Optionally, the report parsing functionality runs during the report generation process, for example optionally as an integrated part thereof.

According to at least some embodiments, the report image interaction supports display related highlighting of a selected image or a portion thereof while the doctor or other medical personnel is generating the report, for example optionally through dictation and/or keyboard or other input device entry. The image interaction module causes the doctor to receive a visual indication as to the selected image or a portion thereof which is to be highlighted, including but not limited to highlighting the image or portion thereof, de-emphasizing other images or other portions of the image that are not to be highlighted, blocking viewing of these other images or other portions of the image that are not to be highlighted, and so forth. All of these types of visual indications are hereinafter referred to as "highlighting".

As used herein, the selected image refers to the image currently being viewed. However, optionally a plurality of images may be currently viewed, or portions of a plurality of images may optionally be currently viewed. The term "focus" refers to the image which is being viewed as the report is prepared. Optionally, more than one image may be viewed simultaneously but typically one image is viewed for the report and hence one image is the subject of the focus.

For example and without limitation, the image interaction module may optionally deliver such information regarding the image under focus to a display device being shown to the doctor. Optionally such information may be delivered to the medical image software which then performs the necessary visual indication being shown by the display device for example by delimiting part of the image.

According to at least some embodiments, the selection of a portion of the image for highlighting may optionally be performed according to segmentation of the image, for example to determine the location and boundaries of one or more organs. Therefore, when the doctor dictates one or more words for such organs, such as for example "liver" or "left lung", segmentation may optionally be used to determine the location and boundaries of the "liver" or "left lung" so that these organs may be highlighted for the doctor. Segmentation may optionally be performed in advance or in real time as the doctor dictates the report. Segmentation may also optionally be performed manually or automatically, or semi-automatically, for example with segmentation software as is known in the art.

Optionally, the image interaction module then delivers information regarding the image or portion thereof that is the subject of each part of the generated report to the report generator, which packages this information together with the generated report itself (which may optionally comprise text, audio, video, other images or a combination thereof) so that when that part of the generated report is being displayed to the report user, the highlighting thereof is visually indicated to the report user.

The term "user" is used herein interchangeably with the terms "doctor", "physician", "radiologist" and the like. All such terms may be understood to refer generally to any user; no limitation is made on the role of the individual who is the user of the various embodiments of the present invention as described herein.

Although the present description relates to interactions with medical image data, it is understood that the present invention may optionally be applied to any suitable image data, including but not limited to computer games, graphics, artificial vision, computer animation, biological modeling (including without limitation tumor modeling) and the like.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.
FIG. 1A shows an exemplary, illustrative system according to at least some embodiments of the present invention for image based report detection of errors for medical image software;
FIG. 1B shows an exemplary, illustrative reporting module from the system shown in Figure 1A, capable of parsing and highlighting image parts according to words found in the report.
FIG. 2 shows an exemplary, illustrative process according to at least some embodiments of the present invention for image based report detection of errors for medical image software, optionally for operating the system and modules of Figures 1A-1B according to at least some embodiments of the present invention; and
FIG. 3 shows an exemplary, illustrative screenshot according to at least some embodiments of the present invention.

### DESCRIPTION OF SOME EMBODIMENTS OF THE INVENTION

Unless otherwise defined, all technical and scientific terms used herein have the same meaning as commonly understood by one of ordinary skill in the art to which this invention belongs. The materials, methods, and examples provided herein are illustrative only and not intended to be limiting.

Implementation of the method and system of the present invention involves performing or completing certain selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

Although the present invention is described with regard to a "computer" on a "computer network", it should be noted that optionally any device featuring a data processor and the ability to execute one or more instructions may be described as a computer, including but not limited to any type of personal computer (PC), a server, a cellular telephone, an IP telephone, a smart phone, a tablet, a PDA (personal digital assistant), or a pager. Any two or more of such devices in communication with each other may optionally comprise a "computer network".

Although the present description relates to medical image data, it is understood that the present invention may optionally be applied to any suitable image data, including but not limited to computer games, graphics, artificial vision, computer animation, biological modeling (including without limitation tumor modeling) and the like.

At least some embodiments of the present invention are now described with regard to the following illustrations and accompanying description, which are not intended to be limiting in any way.

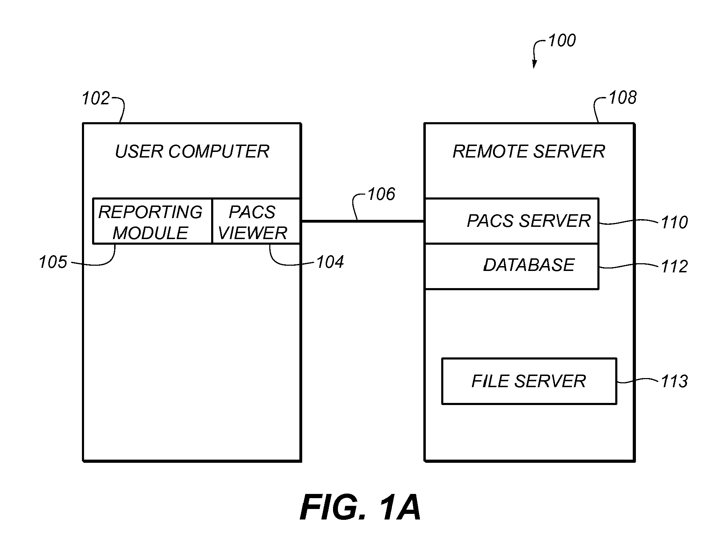

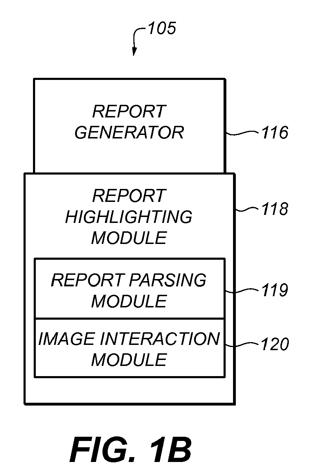

Referring now to the drawings, Figures 1A and 1B show an exemplary, illustrative system according to at least some embodiments of the present invention for image based report detection of errors, with the functionality of report generation and report error detection being distributed over a client/server based configuration. Figures 1A and 1B optionally permit a "zero footprint" implementation but are not limited to this implementation.

Referring to Figure 1A, a system 100 features a user computer 102 that is operated by the user (as used herein, the user is the person generating the report). User computer 102 operates a PACS viewer 104 for viewing images, as a non-limiting example of a software program; any suitable software program or "app" could be used in its place, for example if user computer 102 were to be implemented as a "smartphone" or cellular telephone with computational abilities, or alternatively a web browser, capable of communicating according to HTTP and rendering HTML (HyperText Markup Language) can be used instead.

User computer 102 also operates a reporting module 105, for interacting with PACS viewer 104. As described in greater detail below with regard to Figure 1B, reporting module 105 may optionally comprise any type of functionality for generating a report, including but not limited to voice to text report generation as described in US Provisional Application No. 61/728,993, filed on November 21 2012 and hereby incorporated by reference as if fully set forth herein to the extent necessary to support the embodiment of voice to text report generation. Reporting module 105 supports interaction with the PACS viewer for image based error detection and may also optionally support packaging the report with one or more images, and/or links thereto. However, optionally the report only features text.

User computer 102 is in communication with a remote server 108 through a computer network 106. Computer network 106 may optionally be any type of computer network, such as the Internet for example. It should be noted that remote server 108 may optionally comprise a plurality of processors and/or a plurality of computers and/or a plurality of virtual machines, as is known in the art.

Remote server 108 optionally operates a medical image processing software, shown herein as PACS server 110, although any suitable medical image processing software may optionally be provided, for example which operates according to DICOM standard. PACS server 110 may optionally comprise any type of medical image processing software or a combination of such softwares. PACS server 110 is optionally and preferably in communication with a database 112 for holding medical patient data and with a file server (113) for holding medical image data, which is shown herein as being incorporated into remote server 108 but which may optionally be separate from remote server 108 (not shown).

PACS viewer 104 processes medical image data, for example allowing images to be displayed, manipulated, segmented or otherwise analyzed; supporting "zoom in-zoom out" for different magnifications or close-up views of the images; cropping, highlighting and so forth of the images. PACS viewer 104 enables the user to perform such actions, and to view the results, through user computer 102. For the sake of security, computer network 106 preferably features at least a security overlay, such as a form of HTTPS (secure HTTP) communication protocol, or any type of security overlay to the communication protocol, such as 256-bit SSL3 AES and security certificates for example, and may also optionally feature a VPN (virtual private network) in which a secure "tunnel" is effectively opened between user computer 102 and remote server 108.

Optionally, PACS viewer 104 is implemented through a web browser such that PACS server 110 provides complete support for medical image processing, such that the medical image processing software has "zero footprint" on user computer 102, such that optionally and more preferably not even a "plug-in" or other addition to the web browser is required. Such complete support for remote medical image viewing and analysis is known in the art, and is in fact provided by the Vue Motion product currently being offered as part of Carestream Health offerings. All of these examples relate to examples of "thin clients", with low or "zero" footprints on user computer 102, preferably provided through a web browser but optionally provided through other software.

Reporting module 105 and PACS viewer 104 are optionally combined (not shown).

PACS server 110 may optionally perform segmentation on the medical images, in order for different organs or portions of organs to be highlighted as part of the reporting process (and also to provide the location and boundaries of such organs or portions of organs as part of the report); alternatively and optionally, such segmentation is performed by a different computational device (not shown) and is stored on remote server 108, for example at database 112. Optionally, PACS viewer 104 may optionally perform segmentation.

As shown in Figure 1B, reporting module 105 optionally comprises a report generator module 116, which allows the user to create a report and may optionally be implemented through a currently available reporting application (software). The user interacts with report generator module 116 by either a voice to text engine that allows him to dictate a report, or alternatively or additionally using a keyboard and typing the report. Report generator module 116 optionally and preferably shows the report on the screen, preferably using a word editing type application where the user can see the words that he is dictating or typing and has the options of editing and changing the text being inputted. Reporting module 105 also optionally and preferably comprises a report highlighting module 118, for supporting image based correction as described herein.

Report highlighting module 118 optionally comprises a report parsing module 119 and an image interaction module 120. Report parsing module 119 optionally and preferably detects one or more words provided through report generator 116. For example and without limitation, such words may optionally relate to directionality (right, left, top, bottom, center, etc); organ names or parts thereof (liver, kidney, etc) and other parts of the body or the image to which the doctor could refer. Report parsing module 119 optionally and preferably considers individual words, words in context (50 cc urine left vs the left side), combination of words (left side vs right side), longer phrases (dictionary), and even words/phrases which the doctor adds. Report parsing module 119 may optionally search for the words using straightforward parsing techniques, or may optionally, alternatively or additionally, implement natural language processing type algorithms as is known in the art.

Report parsing module 119 may optionally be configured, e.g. through some configuration file, to search only a subset of the total word and word phrases that it is capable of searching. Alternatively or additionally, it can receive the words to search for from image interaction module 120 as described in greater detail below.

Image interaction module 120 preferably causes the radiologist to receive a visual indication as to the selected image or a portion thereof to be highlighted, including but not limited to highlighting the image or portion thereof, de-emphasizing other images or other portions of the image that are not to be highlighted, blocking viewing of these other images or other portions of the image that are not to be highlighted, and so forth. Optionally, as described in greater detail below, such visual indication is provided through the PACS viewer 104.

Overall, report parsing module 119, image interaction module 120 and the software responsible for displaying the images (PACS viewer 104 in this non-limiting example) may optionally interact as follows. The software responsible for displaying the images preferably communicates which parts of the image can be highlighted (for example according to image segmentation) to image interaction module 120. As non-limiting examples, if the liver has been segmented it can be highlighted in the image, as well as general body locations (for example, the right part of the body), or general image locations such as top half of the image. Image interaction module 120 may then optionally limit the choices of words, word combinations, phrases and so forth available to report parsing module 119.

As the report is being entered, for example through dictation, by the radiologist, report parsing module 119 parses the text at the current cursor position. Optionally, when a match to some word or phrase is found, report parsing module 119 communicates this to image interaction module 120, which then sends a highlighting command to the viewing module (image display software). Highlighting can be changed as the words entered change; for example, if the word "right" is entered and is determined to refer to the right side of the image, then that side is highlighted. If the word "top" is then entered and is determined to refer to the top side of the image, then the top is highlighted and the right side is preferably not highlighted, through the interaction of these modules.

A command for indicating that nothing is to be highlighted is also optionally part of the protocol between report parsing module 119 and image interaction module 120, so that for example whenever the cursor is moved to a position which does not match a word or phrase which needs to be highlighted, this command may be sent from parsing module 119 to image interaction module 120, causing all previously highlighted areas to no longer be highlighted. If, for example, the word "right" is erased, report parsing module 119 detects that the cursor no longer points to a position that should cause highlighting in the image and sends this highlight nothing command to image interaction module 120. Image interaction module 120 communicates this information to PACS viewer 104 which then stops displaying any highlighting currently shown on the image. When a word or phrase is dictated which does relate to a location on the image, such that highlighting is required, a new highlighting command is sent from parsing module 119 to image interaction module 120, and hence to PACS viewer 104, and then the appropriate highlighting is shown again.

For the non-limiting implementation of Figures 1A and 1B, image interaction module 120 may optionally deliver such information regarding the selected image or a portion thereof to be highlighted to PACS viewer 104, causing PACS viewer 104 to alter the rendition of the image being shown to the radiologist through a display device associated with user computer 102. Optionally such information may be delivered to PACS server 110 which then performs the necessary visual indication being shown by the display device.

For example for this implementation, optionally image interaction module 120 communicates with PACS viewer 104, and passes along image manipulation type commands to cause PACS viewer 104 to add or remove highlighting.

Optionally, image interaction module 120 performs segmentation. Optionally, image interaction module 120 does not itself perform segmentation or other image analysis. Instead the image locations that the PACS viewer 104 can highlight or mark are preferably communicated to the image interaction module 120 when a study is loaded, for example from database 112 or PACS server 110. When study is loaded for the purposes of reporting, the image interaction module 120 queries the viewer application (in this example PACS viewer 104) for which type of image interactions and image locations it is capable of supporting. The response to this query include but is not limited to image locations such as left, right, top, bottom, organs or parts of organs that have been segmented through various means and can therefore be highlighted such as left kidney, spleen, liver, left upper lung, liver segment 2 etc.

Report parsing module 119 and image interaction module 120 may optionally operate together as follows. For example, if the current study does not contain a spleen, or the current study does contain a spleen, but PACS viewer 104 cannot segment the spleen (and therefore cannot highlight it), image interaction module 120 may optionally communicate with the report parsing module 119 and instruct the latter not to search for the word "spleen" or related words thereto.

Alternatively, whenever parsing module 119 finds the word "spleen" and communicates this word to image interaction module 120, image interaction module 120 does not further communicate a spleen highlight command to PACS viewer 104 since PACS viewer 104 cannot display highlighting on the spleen for the reasons given above. Alternatively, this knowledge is maintained solely by PACS viewer 104, which in this case optionally makes the ultimate decision whether to highlight a part of the image.

The user preferably interacts with report generator 116 as follows. The user, such as a radiologist for example, reviews medical images through PACS viewer 104, being operated by user computer 102, in communication with remote server 108. As the user reviews these medical images, the user enters a report through user computer 102 while selecting a specific image(s) and/or specific part(s) of specific image(s), for example by entering one of the previously described words, word combinations and/or phrases for parsing by parsing module 119. Information regarding which image(s) or portion(s) thereof is being selected is then provided to image interaction module 120. Image interaction module 120 determines which image(s) or portion(s) thereof is in being selected and transmits the above described visual indication to PACS viewer 104 to highlight on the image(s) or portion(s) under selection. Image interaction module 120 also optionally communicates indications regarding the image(s) or portion(s) thereof that is being selected to report generator 116.

Report generator 116 then optionally packages this information together with the generated report itself (which may optionally comprise text, audio, video, other images or a combination thereof) so that when that part of the generated report is being displayed to the report user, the highlighted image or portion thereof is visually indicated to the report user. As previously described, optionally such indication comprises one or more of highlighting the focused image or portion thereof, de-emphasizing other images or other portions of the image that are not to be highlighted, blocking viewing of these other images or other portions of the image that are not to be highlighted, and so forth.

As an additional option, report generator 116 may also optionally, additionally or alternatively, insert hyperlinks in the text of the generated report to images with the highlighting information as described above.

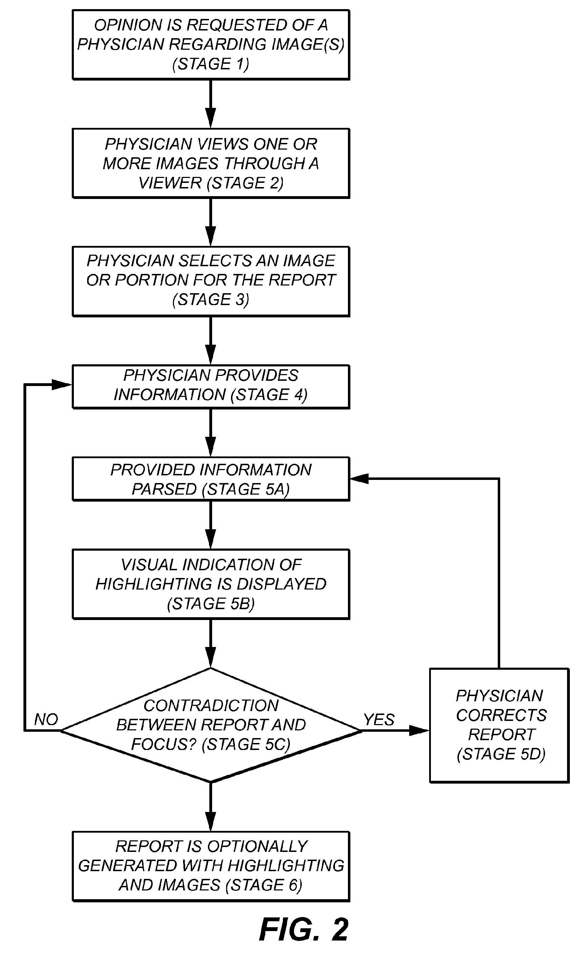

Figure 2 shows an exemplary, illustrative method according to at least some embodiments of the present invention for performing the report generation and error detection method as described herein. As shown, in stage 1, an opinion is requested of a physician regarding an imaging study or alternatively a portion of such a study, comprising one or more images. The request may optionally be sent through a computer network, for example by email, or alternatively may optionally be made verbally.

In stage 2, the physician views one or more images, comprising part or all of an imaging study, according to the request (which may optionally direct the physician to the specific image(s) or study, or alternatively may optionally refer to the patient for example) through a viewing application as described herein, whether a PACS viewer or a "thin client" viewer (for example provided through a web browser as described herein). The viewing application may optionally be provided through a computer or cellular telephone (such as a smartphone) or other electronic device as described herein.

In stage 3, as the physician views the one or more images, the physician selects an image or a portion thereof for description in the report. Optionally, a visual indication in the image or portion thereof being displayed is given to the physician to show focus thereof.

In stage 4, the physician provides information to generate a report, which preferably includes at least text, for example optionally by dictating a verbal (i.e.-voice) report to the electronic device, which is preferably the same electronic device that is displaying the one or more images. Alternatively the physician may enter the information by typing or otherwise entering text.

In stage 5A, the provided information is parsed as previously described, to locate the words, word combinations and/or phrases relating to directionality, parts of the body and so forth corresponding to the current cursor position in the report being entered. In stage 5B, optionally and preferably simultaneously while providing such information, a visual indication of the information referred by the text is displayed to the physician, which may optionally rely upon a previously performed segmentation and includes highlighting the relevant parts of the image.

In stage 5C, if no contradiction is found by the user between the pathology being reported and the image or portion thereof under highlighting, then the process returns to stage 4 and more information is entered.

However, if in stage 5C, the physician determines that the highlighted area is not in actuality the area that the physician meant, then the process optionally continues as shown. For example, the physician may have meant to say the left side of the image has an injury, yet may mistakenly state that the injury is on the right side. In this case, the right side is highlighted as the visual indication, and the physician is able to detect the contradiction between the word(s) in the entered information and the injury appearing in the non-highlighted part of the image. The physician may also optionally request to have the report read back, whether audibly or by highlighting different parts of the text. This part of the process may also optionally be performed after error correction and/or after the report is generated. In any case, in stage 5D, the report is corrected and the process returns to stage 5A.

In stage 6, optionally, when the physician finishes dictating or writing the report, the report is generated by packaging the entered text, the one or more images, or image study, and the highlighting information, thereby enabling the opinion and thoughts of the physician to be captured and to be made part of the permanent record regarding the image(s) viewed.

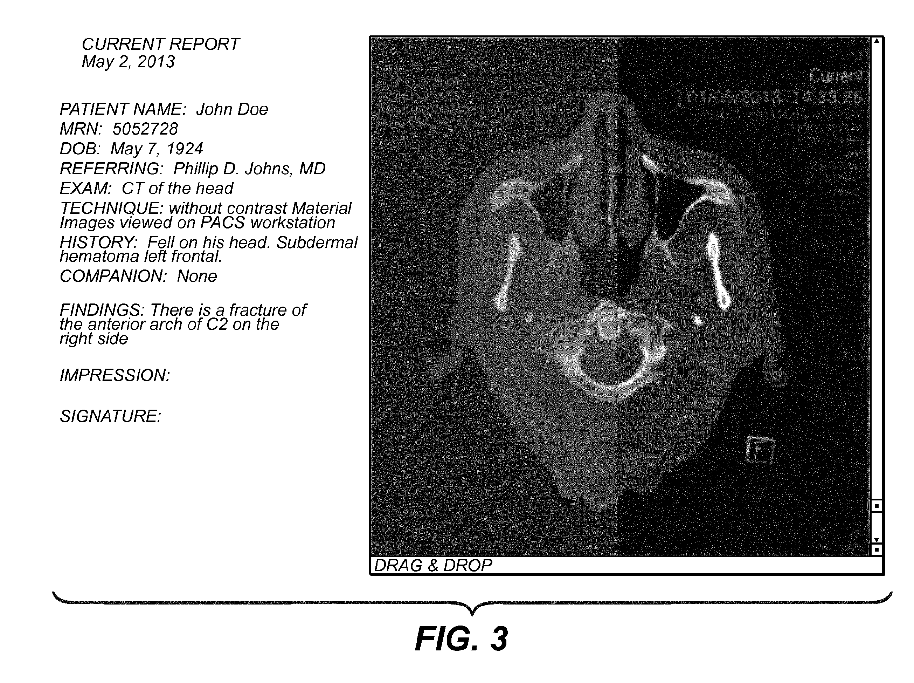

Figure 3 shows an exemplary, illustrative screenshot according to at least some embodiments of the present invention. The left side of the image is being highlighted since the physician dictated a fracture on the anterior arch of C1 on the right side (note that in radiological images the left side of the image corresponds to the right side of the body), yet the fracture is actually on the left side as can be seen in the image. The radiologist realizes that the software does not highlight the actual fracture and is able to easily detect his mistake using the visual queue of the highlighting. He then proceeds to correct the word right to the word left whereby the image will be highlighted on its right side, highlighting the fracture.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A system for report image interaction, comprising: a medical image software for rendering medical images, a user electronic device comprising a display for displaying the images, and a reporting module for receiving report information and for communicating with the medical image software to highlight an image or a portion thereof from the report information.

2. The system of claim 1, wherein the reporting module comprises a report generator for generating the report, and a report highlighting module for parsing the report information and for communicating with the medical image software.

3. The system of claim 2, wherein the report highlighting module comprises a report parsing module and the report parsing module parses the report information according to individual words, combinations of words, words in context or phrases, or a combination thereof; wherein the words indicate one or more of directionality or a part of a body.

4. The system of claim 2, wherein the report generator generates the report, the report comprising one or more images, information regarding highlighting associated with each image or image portion and the report information.

5. The system of claim 4, wherein the report generator further comprises a voice to text engine for receiving voice data and for converting the voice data to words for the report information.

6. The system of claim 4, wherein the report generator further comprises text input and editing functions.

7. The system of claim 1, wherein the reporting module further comprises an image interaction module for communicating with the display to indicate the highlighting, wherein the highlighting is selected from the group consisting of highlighting the selected image or portion thereof, de-emphasizing other images or other portions of the image that are not to be highlighted and blocking viewing of these other images or other portions of the image that are not to be highlighted.

8. The system of claim 7, wherein the image interaction module determines which word(s) or phrase(s) are available to the reporting parsing module according to the highlighting.

9. The system of claim 7, wherein the image interaction module removes existing highlighting and blocks highlighting for a particular word, words, phrase or phrases if the image interaction module determines that highlighting is not possible for the word, words, phrase or phrases.

10. The system of claim 7, wherein the image interaction module performs segmentation on the image to determine a location and boundaries of one or more organs to enable the highlighting on a portion of the image.

11. A method for image based error detection, the method being performed by a computer, comprising:
providing medical image software for rendering medical images;
displaying one or more medical images;
receiving report information regarding a medical image or portion thereof, by the computer, the report information comprising a plurality of words;
parsing the plurality of words by the computer to determine one or more of directionality or a part of a body of the image or portion thereof; and
highlighting a portion of the medical image according to highlighting determined by the parsing by the computer.

12. The method of claim 11, further comprising displaying the highlighting and the medical image through a display.

13. The method of claim 11, further comprising receiving a correction to the report information, comprising:
changing one or more words of the report information to form changed words;
parsing the changed words to determine one or more of directionality or a part of a body of the image or portion thereof;
determining that a change in highlighting of a portion of the medical image is required according to the parsing of the changed words by the computer; and
displaying changed highlighting.

14. The method of claim 11, wherein the display comprises a PACS viewer, and the method further comprising querying the PACS viewer to determine which type of image interaction or interactions, and image location or locations, the PACS viewer is able to support.

15. The method of claim 11, further comprising removing existing highlighting and blocking highlighting for a particular word, words, phrase or phrases if highlighting is not possible for the word, words, phrase or phrases.

16. The method of claim 11, further comprising generating a report by a report generator operated by the computer, the generating the report comprising packaging the one or more images and the highlighting for each image, and the report information, into the report.

17. The method of claim 11, wherein highlighting of the portion of the medical image according to the highlighting determined by the parsing further comprises one of: determining segmentation of the medical image or receiving the segmentation of the medical image; determining a location and boundaries of at least one organ according to the segmentation; and applying the highlighting to at least a portion of the medical image according to the location and boundaries of the at least one organ.
